# EUROPEAN PATENT APPLICATION

(11) **EP 3 854 348 A1**
(43) Date of publication of application: **28.07.2021**
(21) Application number: 20315006.5
(22) Date of filing: 23.01.2020
(51) Int. Cl.: A61F 2/06, A61F 2/00

(54) **ENDOPROSTHESIS AND METHOD OF ATTACHING A FIBER TO A SURFACE**

(71) Applicant: Kardiozis SAS, 13100 Aix-en-Provence (FR)
(72) Inventor: VANDAELE FENOUIL, Nathalie, 13100 Aix-en-Provence (FR); BURG, Brian, 13100 Aix-en-Provence (FR); CHAPUT, Oriane, 13100 Aix-en-Provence (FR)
(74) Representative: Hepp Wenger Ryffel AG

(57) **Abstract**

The invention relates to an endoprosthesis (60), in particular a vascular stent or a heart stent, comprising a graft (2) with a graft surface (3). The endoprosthesis (60) further comprises at least one fiber (1) which is arranged on the graft surface (3). The graft surface is adapted to directly attach the fiber (1).

## Description

The present invention relates to an endoprosthesis and a method of attaching a fiber to a surface according to the preamble of the independent claims.

Endoprostheses, in particular vascular or heart stents, are used to support blood vessels in the human body. For example, occlusions or aneurysms can be treated by placing such an endoprosthesis at the respective treatment site. In the treatment of an occlusion, the endoprosthesis keeps the vessel open for unhindered blood flow. In the case of an aneurysm, the endoprosthesis can prevent circulation of blood into the aneurysm and thus lower the risk of a thrombus, rupture or further growth of the aneurysm.

It is known in the prior art to use thrombogenic elements on endoprostheses. For example, WO 2013/182614 A1 discloses an endoprosthesis with thrombogenic elements that extend away from a body of the endoprosthesis and promote thrombosis. This allows for the occlusion of an aneurysm to enhance the above-mentioned treatment effect.

WO 2006/0166167 discloses fibers arranged on an endovascular prosthesis.

WO 2019/122944 discloses the attachment of a strip of fabric with fibers on a stent graft.

However, currently known methods do not provide a simple way of post-production arrangement of thrombogenic elements on an existing endoprosthesis. Fixation and attachment of thrombogenic elements is usually cumbersome and difficult, and not typically versatile. In addition, known mechanisms are limited to generic thrombus generation means that are not adapted to patient-specific needs.

Thus, the object of the present invention is to overcome the drawbacks of the prior art, in particular to provide an endoprosthesis and a method to produce an endoprosthesis wherein thrombogenic elements can easily be added to a surface of the endoprosthesis, in particular in a versatile manner and at selected locations on the endoprosthesis surface. The endoprosthesis also may be suitable for subsequent attachment of thrombogenic elements, in particular depending on patient specific needs.

This and other objects are achieved by the endoprosthesis and the method according to the characterizing portion of the independent claims.

The endoprosthesis according to the invention may in particular be a vascular stent or a heart stent. It comprises a graft with a graft surface. The endoprosthesis further comprises at least one fiber which is arranged on the graft surface. The graft surface is adapted to attach the fiber. In particular, the graft surface is adapted such that a fiber can be directly attached to the graft surface without an additional support structure. The graft surface may be adapted to directly attract the fiber material, for example through chemical or physical bonding, or through mechanical attachment such as friction. This is particularly advantageous in that fibers can be attached to the surface individually and with a high degree of flexibility. For example, the fiber may be attached with a free end to the graft surface, or it may be attached in a middle section such that two free ends remain and extend away from the surface. Alternatively or additionally, however, it is also possible to adapt the fibers such that they comprise an anchoring element that can interact with the graft surface and/or an attachment mechanism on the graft surface.

Preferably, at least one of the fiber or the graft has a surface that is adapted such that the fiber is retained on the graft surface and/or a graft material by friction. This provides for a particularly easy attachment of a fiber because no additional steps or materials are necessary. Friction may be enhanced by material selection, i.e. by providing a material and/or surface coating in the graft surface that has a high friction coefficient on a fiber material and/or fiber surface. Additionally or alternatively, the graft surface and/or the surface of the fiber may be structured to enhance friction. It is also conceivable to treat the surface to enhance electrostatic or magnetic interactions. A high degree of friction between the fiber and the graft is particularly advantageous if the fiber is sewn in the graft as the friction prevents displacement of the fiber and thus makes additional retention mechanisms such as knots and glues unnecessary.

Preferably, the graft surface is coated with an adhesive material that is adapted to provide adhesion the surface of the fiber. For example, a glue may be coated on the graft surface. The graft material could also be coated with polycaprolactone (PCL). The glue may be selected according to the materials comprised in the graft and the fiber. In addition, the glue is preferably biocompatible. Additionally or alternatively, adhesion may also be provided by electrostatic forces, magnetic forces, and/or chemical bonds. The adhesive material can be activatable such that adhesion to the fibers is only provided after activation through an activation mechanism. Alternatively, the adhesive layer may be non-activatable.

Particularly preferably, the adhesive material is bioactive. The bioactive material could be selected of the group consisting of enzymes, proteins, DNA, RNA, antibodies or antigens.

Preferably, the adhesive material is adapted such that it is activatable by at least one of heat, pressure, chemical substances (e.g. oxygen or water) and radiation. For example, the adhesive material may comprise a hot melt adhesive that can be molten by heat. Such an adhesive material enables easy attachment of fibers in the molten adhesive material, but safe attachment in a solid state. In addition, misplacement of fibers may be corrected by re-heating. The adhesive material could also be a pressure-sensitive adhesive as known in the art. Additionally or alternatively, it is also conceivable to use an adhesive material that can be activated by exposure to electromagnetic radiation such as UV light, IR light, visible light, microwave radiation, or ultrasonic waves.

Preferably, the adhesive material is coated on discrete positions. This shall in particular encompass islands of adhesive material and/or other portions of adhesive material that are not connected to each other, i.e. do not continuously cover the graft surface. Particularly preferably, a small portion of adhesive material are placed on the graft surface at a position where a fiber shall be placed.

Preferably, the fiber comprises a material which is, in a molten or partially molten state, at least partially miscible with a material comprised by the graft and/or the graft surface. This enables an attachment of the fiber on the graft and/or the graft surface through partial melting. For example, the fiber may be placed on the graft surface and heated up such that the graft surface and the fiber partially melt. Due to their miscibility, a permanent attachment of the fiber to the graft surface is achieved upon cooling. This enables, for example, an attachment through welding.

The graft may also comprise a mechanical structure that is adapted to be attached to a fiber. Particularly preferably, the mechanical structure is selected from a group comprising a hole, a loop, and a rivet. Any form fit collection might be suitable. Such mechanical structures can enable a reduction of the number of materials used and thus make manufacturing easier. It can be possible to configure the mechanical structure to comprise or consist of the same material as the fiber and/or the graft. Using mechanical structures also reduces the need to expose the graft to radiation, chemicals, and other treatment steps and reduce manufacturing errors.

It will be understood by the person skilled in the art that any of the above attachment mechanisms could be combined with one another. For example, the attachment of a fiber with a rivet does not preclude an additional application of an adhesive and/or partial melting.

The invention further relates to a method of attaching a fiber to a surface. In particular, the surface may be a surface of a graft for an endoprosthesis. The method is particularly suited to attach a fiber to an endoprosthesis as disclosed herein. The method comprises a step of providing a graft that comprises a graft surface. A fiber is attached to the surface. The attachment is performed by at least one of welding, gluing, heating by exposure to electromagnetic radiation, and mechanical attachment.
The method typically is carried out as a separate step after manufacturing so that e.g. individual characteristic of a patient may be taken into account.

The invention further relates to a catheter with an endoprosthesis as described above, detachably mounted on a holder of the catheter.

In the following, the invention is described in detail with reference to the following figures, showing:
- Fig. 1:: a first embodiment of a graft surface with an attached fiber.
- Fig. 2:: a second embodiment of a graft surface with an attached fiber.
- Fig. 3:: a third embodiment of a graft surface with an attached fiber.
- Fig. 4:: a fourth embodiment of a graft surface with an attached fiber.
- Fig. 5:: a fifth embodiment of a graft surface with an attached fiber.
- Fig. 6:: a sixth embodiment of a graft surface with an attached fiber.
- Fig. 7:: a seventh embodiment of a graft surface with an attached fiber.
- Fig. 8:: an eighth embodiment of a graft surface with an attached fiber.
- Fig. 9:: an endoprosthesis with attached fibers on a graft surface.

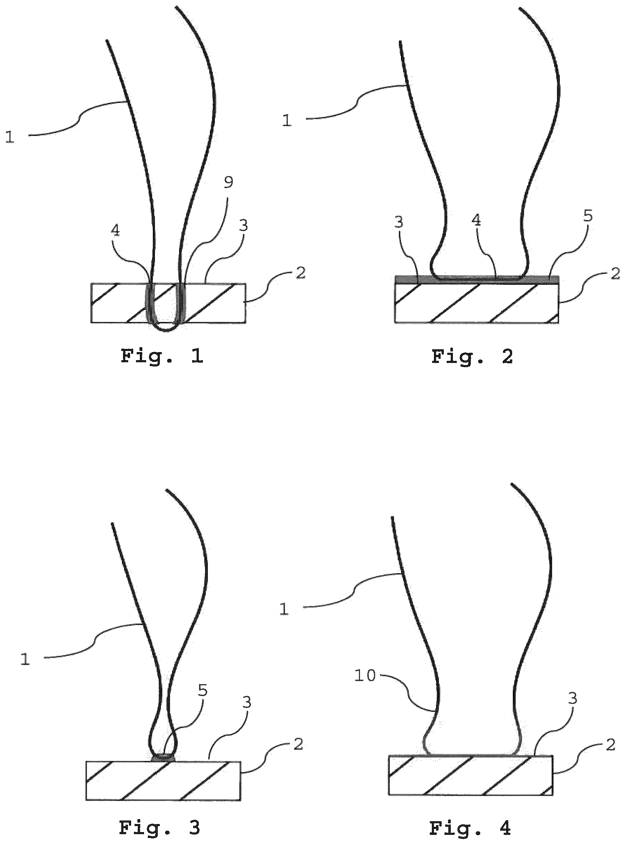

Fig. 1 shows a first embodiment of a graft 2 with a graft surface 3 adapted to attach a fiber 1. In the shown embodiment, the graft 2 consists of a biocompatible Dacron fabric with a thickness of 0.1 mm. Alternatively, the graft may have any thickness in the range of 0.05 to 0.2 mm. The fiber 1 consists of the same material and has a total length of 2 mm. Alternatively, the fiber may up to 50 mm in length. The fiber 1 was stitched through the graft 2. The surface 4 of the fiber 1 is thus arranged in a channel 9 within the graft 2. The channel 9 has an inner surface that is not specifically treated and therefore is substantially identical to the graft surface 3. The graft surface 3 within the channel 9 or on the side opposite the fiber 1 provides sufficient friction with the surface 4 of the fiber 1 such that the fiber 1 cannot be longitudinally displaced within the channel 9. Thus, the fiber 1 is attached to the graft 2 without the need for any additional mechanisms such as gluing, melting, or welding. It would, however, be possible to use such an additional mechanism for increased attachment. Additionally or alternatively, the fiber 1 and/or the graft surface 3, in particular within the channel 9, could be additionally modified and provided with a surface structure to enhance friction.

Fig. 2 shows an alternative embodiment wherein the graft 2 has been provided with an adhesive material 5 on the graft surface. The adhesive material 5 is a hot melt glue based on polyethylene terephthalate and is applied on the graft surface 3 with a thickness of 40 µm. The fiber 1, which in the shown embodiment consists of polyethylene terephthalate with a higher molecular mass than the hot melt, was arranged on the adhesive layer 5. Heating temporarily liquified the hot melt adhesive material 5 and allowed the fiber 1 to be incorporated in the adhesive material 5. Because the fiber 1 has a higher molecular mass than the adhesive material 5, the temperature for heating can be selected such the adhesive material 5 melts, but not the fiber 1. However, because both consist of the same polymer, the wetting between the two surfaces 3,4 is sufficient for the fiber 1 to be incorporated in the adhesive material 5. Upon cooling, the fiber 1 is fixedly attached in the adhesive material.

Fig. 3 shows an embodiment where an adhesive material 5 is only placed on the graft surface 3 at a discrete location. As such, the attachment of the fiber 1 is similar to the embodiment of Fig. 2. However, the fiber 1 can only be attached at the position where the adhesive material 5 is located. In the shown embodiment, the adhesive material 5 is based on an avidin protein. The fiber 1 is functionalized with a biotin protein. By pressing the fiber 1 on the adhesive material 5, the fiber 1 is strongly attached to the adhesive material 5 by forming an avidin-biotin complex. The adhesive-material further comprises a bioactive substance, in this case an enzyme. Alternatively, the bioactive substance may also be a protein, DNA, RNA, antibodies or antigens.

Fig. 4 shows an alternative embodiment. The fiber 1 consists of isotactic polypropylene. The graft 2 is made of a material that is miscible with isotactic polypropylene. Thus, the fiber 1 placed on the graft surface 3 will form an integral connection with the graft if both are partially melted. Thus, the fiber 1 is connected to the graft 2 by heating up to approximately 200°C.

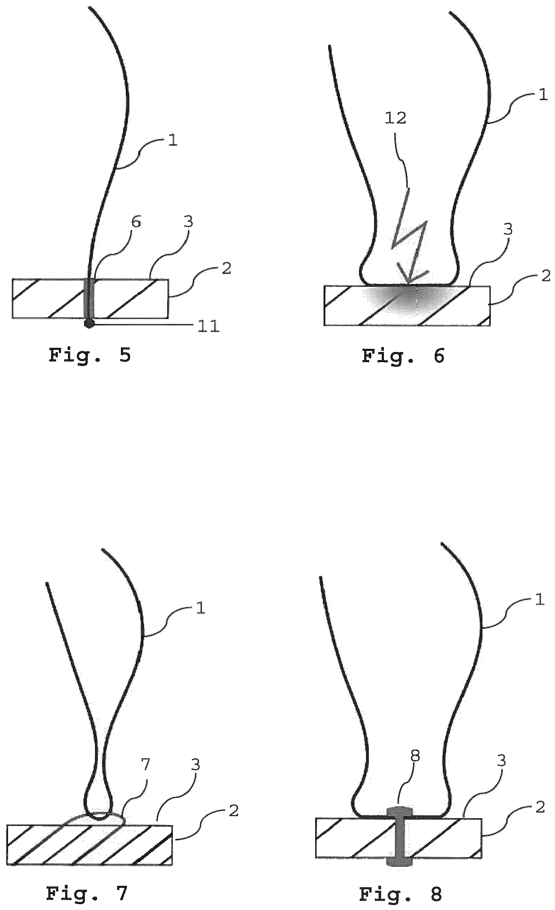

Fig. 5 shows an alternative embodiment wherein the graft 2 is provided with a hole 6. The fiber 1 further comprises a retention ball 11 arranged at a free end. The fiber 1 is arranged within the hole 6. The retention ball 11 has a larger diameter than the hole 6 and thus provides a form-fit connection and prevents the fiber 1 from being removed from the hole 6. Additionally or alternatively, it would also be possibly to arrange an element with a different shape, such as a disk, a cross, an egg-shape, or any other shape that provides retention in the hole 6.

Fig. 6 shows an embodiment of a fiber 1 that is attached to the graft surface 3 by exposure to radiation 12. Here, the graft 2 has a functionalized graft surface 3 that allows for chemical cross-linking. The fiber 1 is made of a cross-linkable polymer. Thus, the fiber 1 can be placed on the graft surface 3 and irradiated with electromagnetic radiation 12, for example UV light. As a consequence of the exposure to radiation, the fiber 1 is chemically cross-linked with the graft surface 3 and permanently attached thereto, corresponding to an activatable adhesive that is adapted to be activated by exposure to radiation.

Fig. 7 shows an alternative embodiment of a fiber 1 being attached to a graft 2 by means of a mechanical attachment. The graft surface 3 comprises a loop 7. The loop 7 is made by a separate thread sewn into the graft 2. The fiber 1 is arranged within the loop 7 and held within the loop 7 by friction. It would also be conceivable to further attach the fiber 1 by means of an adhesive, or to provide fiber 1 with a knot.

Fig. 8 shows a further alternative embodiment of a fiber 1 being attached to a graft 2 by a mechanical attachment. Here, a rivet 8 is arranged through the graft 2 and fixes the fiber 1 on the graft surface 3.

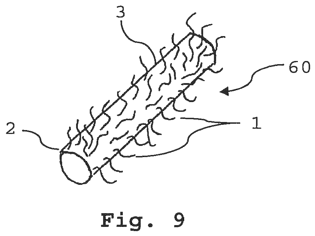

Fig. 9 shows an exemplary endoprosthesis 60 according to the invention. The endoprosthesis comprises a scaffold formed by a stent known to the skilled person and carrying the graft material. Several fibers 1 are arranged on the graft surface 3 of the graft 2. Any of the above-mentioned attachment mechanisms is suitable to attach the fibers 1 to the graft surface 3. In the present embodiment, all the fibers are attached by the same mechanism. However, it would also be conceivable to attach different fibers 1 with different mechanisms and thus include several of the above-described mechanisms in one endoprosthesis. In particular, fibers with varying lengths between 2 and 50 mm may be attached to the graft surface by the above-described mechanisms. Similarly, the present embodiment shows a plurality of fibers 1 attached on the graft surface 2 with a substantially uniform distribution. The person skilled in the art will understand that this is merely an exemplary embodiment and that any number of fibers 1 could be attached to the graft surface 3, or with any distribution desired for a particular application. The endoprosthesis may be self-expandable and/or balloon expandable. It can be deployed by means of a catheter in a minimally invasive manner and can be fixed to the catheter.

## Claims

1. An endoprosthesis (60), in particular a vascular stent or a heart stent, comprising a graft (2) with a graft surface (3), further comprising at least one fiber (1) arranged on the graft surface (3), **characterized in that** the graft surface is adapted to attach the fiber (1).

2. Endoprosthesis (60) according to claim 1, wherein at least one of the graft (2) or fiber (1) has a surface (4) that is adapted such that the fiber (2) is retained on the graft surface (3) and/or a graft material by friction.

3. Endoprosthesis (60) according to one of the preceding claims, wherein the graft surface (3) is at least partially coated with an adhesive material (5) that is adapted to provide adhesion to the surface (4) of the fiber (1).

4. Endoprosthesis (60) according to claim 3, wherein the adhesive material is bioactive or comprises a bioactive material.

5. Endoprosthesis (60) according to one of the claims 3 or 4, wherein the adhesive material (5) is adapted such it is activatable by at least one of heat, pressure, and radiation.

6. Endoprosthesis (60) according to one of the claims 3 to 5, wherein the adhesive material (5) is coated on discrete positions of the graft surface (3).

7. Endoprosthesis (60) according to one of the preceding claims, wherein the fiber (1) comprises a material which is, in a molten or partially molten state, at least partially miscible with a material comprised by the graft (2) and/or the graft surface (3).

8. Endoprosthesis (60) according to one of the preceding claims, wherein the graft (2) comprises a mechanical structure (6,7,8) that is adapted to be attached to a fiber (1), preferably a mechanical structure selected from a group comprising a hole (6), a loop (7), and a rivet (8).

9. A method of attaching a fiber (1) to a surface, preferably a surface (3) of a graft (2) for an endoprosthesis (60), in particular an endoprosthesis according to one of the preceding claims, wherein a graft (2) comprising a graft surface (3) is provided, and a fiber (1) is attached to the surface, **characterized in that** the attachment of the fiber is performed by at least one of welding, gluing, heating by exposure to ultrasound waves, heating by exposure to electromagnetic radiation, and mechanical attachment.

10. The method according to claim 9, wherein the fiber is attached to the surface in a separate step after manufacturing the endoprosthesis.

11. A catheter including an endoprosthesis according to one of the claims 1 to 8, wherein the endoprosthesis is detachably mounted to a holder of the catheter.
